# EUROPEAN PATENT SPECIFICATION

(11) **EP 1 998 743 B1**
(45) Date of publication and mention of the grant of the patent: **26.06.2013**
(21) Application number: 06751298.8
(22) Date of filing: 20.04.2006
(51) Int. Cl.: A61K 8/55, A61Q 11/00

(54) **Oral care compositions providing enhanced whitening and stain prevention**
Mundpflegezusammensetzungen für schöneres Weiss und besseren Schutz vor Verfärbungen
Compositions de soins bucco-dentaires assurant un blanchiment et une prévention des taches améliorés

(30) Priority: 29.03.2006 US 391838
(43) Date of publication of application: 10.12.2008
(73) Proprietor: The Procter & Gamble Company, Cincinnati, OH 45202 (US)
(72) Inventor: SCOTT, Douglas, Craig, Loveland, Ohio 45140 (US); GLANDORF, William, Michael, Mason, Ohio 45040 (US); DRAKE, Phillip, Asa, Mason, Ohio 45040 (US)
(74) Representative: Joos, Uta Susanne
(86) International application number: PCT/US2006/015536
(87) International publication number: WO 2007/111616

(56) References cited:
- WO-A-98/22080
- WO-A2-2004/039277
- FR-A- 1 559 617
- US-A- 5 939 052
- US-B1- 6 713 049

## Description

### TECHNICAL FIELD

The present invention relates to mouthrinse compositions containing an agent having substantivity to teeth, namely polyphosphorylated compounds selected from linear condensed polyphosphate polymers having an average chain length of 6 to 21 and phytate, which in combination with a tooth bleaching agent such as peroxide provide enhanced whitening and stain prevention of teeth.

### BACKGROUND OF THE INVENTION

Oral care products such as dentifrice and mouthrinse are routinely used by consumers as part of their oral care hygiene regimens. It is well known that oral care products can provide both therapeutic and cosmetic hygiene benefits to consumers. Therapeutic benefits include caries prevention which is typically delivered through the use of various fluoride salts; gingivitis prevention by the use of an antimicrobial agent such as triclosan, stannous fluoride, or essential oils; or hypersensitivity control through the use of ingredients such as strontium chloride or potassium nitrate. Cosmetic benefits provided by oral care products include the control of plaque and calculus formation, removal and prevention of tooth stain, tooth whitening, breath freshening, and overall improvements in mouth feel impression which can be broadly characterized as mouth feel aesthetics. Calculus and plaque along with behavioral and environmental factors lead to formation of dental stains, significantly affecting the aesthetic appearance of teeth. Behavioral and environmental factors that contribute to teeth staining propensity include regular use of coffee, tea, cola or tobacco products, and also the use of certain oral products containing ingredients that promote staining, such as chlorhexidine and stannous salts.

While the art has addressed some of the formulation issues of oral care products relating to cosmetic benefits, there continues to be a need in the area, particularly in Whitening and stain prevention from products for daily use such as dentifrice and mouthrinse.

The tooth structures that are generally responsible for presenting a stained appearance are enamel, dentin, and the acquired pellicle. Extrinsic staining of the acquired pellicle can arise as a result of compounds, such as tannins and other polyphenolic compounds that have become trapped in and tightly bound to the proteinaceous layer on the surface of the teeth. Discoloration from this type of staining can usually be removed by mechanical methods of tooth cleaning. In contrast, intrinsic staining occurs when the staining compounds penetrate the enamel and even the dentin, or alternatively, such staining arises from sources within the tooth. Discoloration from intrinsic staining is not readily amenable to mechanical methods of tooth cleaning. Chemical methods, which utilize substances that can penetrate into the tooth structure, are usually required to eliminate such discoloration. Thus, for oral care products for daily use such as dentifrice and rinses to provide overall cleaning, it is necessary to add ingredients for provision of antiplaque and anticalculus benefits as well as stain removal, stain control and tooth whitening. Such ingredients for removal and control of stain and for whitening include abrasives for mechanical cleaning and bleaches, surfactants and chemical chelants for chemical cleaning. Dental abrasives provide important whitening benefits, particularly on 'brushed' areas of teeth, but unfortunately are of limited effect in controlling aesthetically undesirable stains that form along the gumline and interproximally. The stain is mechanically abraded through the use of abrasives or polishing agents normally employed in toothpaste preparations. Bleaches such as urea peroxide, hydrogen peroxide or calcium peroxide, represent the most common forms of whitening agents for teeth. It is believed that peroxides whiten teeth by releasing hydroxyl radicals capable of breaking down the plaque/stain complex into a form that can be flushed away or removed by an abrasive. However, bleaches added to dentifrice and mouthrinse are typically present in low concentrations due to stability and safety limits unique to these product types. At these low concentrations, bleaches which are oxidizing agents, have not generally been effective at tooth whitening and stain control. Bleaches and abrasives do not functionally act to prevent acquisition of stains. Abrasive use can reduce rates of stain acquisition by daily removal of newly acquired stains, but this action is a 'treatment' for existing stain, not a preventive chemical action.

Chelants have been suggested in the art for the purpose of retarding calculus formation and removing calculus after it is formed. The chemical approach to calculus inhibition generally involves chelation of calcium ion and/or crystal growth inhibition which prevents the calculus from forming and/or breaks down mature calculus by removing calcium. In addition, chemical chelants can in principle remove stains by binding to teeth surfaces thereby displacing color bodies or chromagens that cause staining. The retention of these chelants can also prevent stains from accruing due to disruption of binding sites of color bodies on tooth surfaces.

A number of agents with chelating properties for use in controlling plaque, calculus and stain have been disclosed in the art. For example, ethylenediaminetetraacetic acid, nitrilotriacetic acid and related compounds are disclosed in British Patent 490,384, Feb. 15, 1937; polyphosphonates in U.S. Pat. No. 3,678,154, Jul. 18, 1972 to Widder et al., U.S. Pat. No. 5,338,537 issued to August 16, 1994 to White, Jr., and US Pat. No. 5,451,401 issued Sep. 19, 1995 to Zerby et al.; carbonyl diphosphonates in U.S. Pat. No. 3,737,533, Jun. 5, 1973 to Francis; a zinc-polymer combination formed by the reaction or interaction of a zinc compound with an anionic polymer containing carboxylic, sulfonic and/or phosphonic acid radicals in U.S. Pat. No. 4,138,477, issued Feb. 6, 1979, to Gaffar; tartaric acid in U.S. Patent Nos. 5,849,271 issued December 15, 1998 and 5,622,689 issued April 22, 1997 both to Lukacovic; acid or salt form of tartrate monosuccinate, tartrate disuccinate, and mixtures thereof in U.S. Pat. No. 5,015,467 issued May 14, 1991 to Smitherman; acrylic acid polymer or copolymer in U.S. Pat. No. 4,847,070, July 11, 1989 to Pyrz et al. and in U.S. Pat. No. 4,661,341, Apr. 28, 1987 to Benedict et al.; sodium alginate in U.S. Pat. No. 4,775,525, issued Oct. 4, 1988, to Pera; polyvinyl pyrrolidone in GB 741,315 published November 30, 1955, WO 99/12517 published March 18, 1999 and U.S. Pat. Nos. 5,538,714 issued July 23, 1996 to Pink et al.; and copolymers of vinyl pyrrolidone with carboxylates in U.S. Patent Nos. 5,670,138 issued Sep. 23, 1997 to Venema et al. and in JP Publication No. 2000-0633250 to Lion Corporation, published February 29, 2000.

Dentrifrices and mouthwashes containing soluble pyrophosphate salts have also been disclosed in the art, the pyrophosphates being indicated for a variety of purposes including as anticalculus agent. Included among such disclosures are U.S. Pat. No. 2,941,926, Jun. 21, 1960 to Salzmann et al.; U.S. Pat. Nos. 3,927,201 and 3,927,202, Dec. 16, 1975 to Baines et al. and Harvey et al., respectively; U.S. Pat. Nos. 4,244,931, Jan. 13, 1981 and 4,247,526, Jan. 27, 1981 to Jarvis et al.; Japanese Patent Application No. 4945-1974; U.S. Pat. Nos. 4,323,551 issued Apr. 6, 1982, 4,515,772 issued May 7, 1986 and 4,885,155 issued December 5, 1989 to Parran et al.; and U.S. Pat. No. 4,822,599 issued April 18, 1989 to Mitra. Also Draus, Lesniewski and Miklos disclose the in vitro effectiveness of soluble pyrophosphate salts against calculus in "Pyrophosphate and Hexametaphosphate Effects in Vitro Calculus Formation", Arch. Oral Biol., Vol. 15, pp. 893-896, (1970).

Linear molecularly dehydrated polyphosphate salts for use as calculus inhibitor are disclosed in U.K. Patent Application GB 2,200,551, Gaffar, Nabi and Jannone, filed Jan. 27, 1988, published Aug. 10, 1988; and in U.S. Pat. No. 4,627,977, issued Dec. 9, 1986, to Gaffar et al. Included among the salts is sodium tripolyphosphate (STPP). Other references disclosing STPP include U.S. Pat. No. 4,923,684, May 8, 1990 to Ibrahim et al. and U.S Pat. Nos. 5,096,701 issued March 17, 1992 and 5,176,900 issued January 5, 1993 both to White et al.

Document WO-A-9304664 discloses mouthrinse compositions having a pH value in the range from 3.5 to 4.5 and comprising hydrogen peroxide.

Commonly-assigned U.S. Patent Nos. 5,939,052; 6,350,436; 6,667,027 and 6,713,049 describe stable oral formulations containing certain surface-active polymers such as linear condensed polyphosphate polymers in combination with a fluoride source. Detailed studies of the therapeutic and cosmetic benefits of these formulations have revealed surprising levels of fluoride anticaries efficacy, along with improved efficacy in calculus and stain prevention. Research has now revealed that such polymeric mineral surface-active agents (PMSA), in particular linear condensed polyphosphates combined with bleaching agents such as peroxides in daily use products such as dentifrice and mouthrinse provide enhanced teeth whitening and stain prevention. It has also been found that polyphosphorylated compounds such as phytic acid can be used instead of or in addition to the linear condensed polyphosphate. The formulations are surprisingly stable and pleasant tasting, thereby encouraging user compliance and daily use.

### SUMMARY OF THE INVENTION

The present invention is directed to oral care compositions in the form of a mouthrinse according to claim 1 providing enhanced teeth whitening and stain prevention, comprising a tooth bleaching active such as a peroxide source and an agent having substantivity to teeth, namely polyphosphorylated compounds selected from linear condensed polyphosphate polymers having an average chain length of 6 to 21 and polyphosphorylated inositol compounds such as phytic acid, myo-inositol pentakis(dihydrogen phosphate), myo-inositol tetrakis(dihydrogen phosphate), myo-inositol trikis(dihydrogen phosphate). The compositions are surprisingly stable against significant loss of peroxide for a period of at least three months under accelerated aging conditions at about 40 °C. Importantly, the compositions are stable against significant flavor degradation and maintain a pleasant taste, thereby encouraging user compliance and frequent use.

The present compositions have a pH ranging from 4.0 to 6 to maintain peroxide stability as well as minimize polyphosphate hydrolysis, which becomes significant below pH 4.

These and other features, aspects, and advantages of the present invention will become evident to those skilled in the art from the detailed description which follows.

### DETAILED DESCRIPTION OF THE INVENTION

While the specification concludes with claims particularly pointing out and distinctly claiming the invention, it is believed that the present invention will be better understood from the following description.

All percentages and ratios used hereinafter are by weight of total composition, unless otherwise indicated. All percentages, ratios, and levels of ingredients referred to herein are based on the actual amount of the ingredient, and do not include solvents, fillers, or other materials with which the ingredient may be combined as a commercially available product, unless otherwise indicated.

All measurements referred to herein are made at 25°C unless otherwise specified.

Herein, "comprising" means that other steps and other components which do not affect the end result can be added. This term encompasses the terms "consisting of" and "consisting essentially of."

As used herein, the word "include," and its variants, are intended to be non-limiting, such that recitation of items in a list is not to the exclusion of other like items that may also be useful in the materials, compositions, devices, and methods of this invention.

As used herein, the words "preferred", "preferably" and variants refer to embodiments of the invention that afford certain benefits, under certain circumstances. However, other embodiments may also be preferred, under the same or other circumstances.

By "oral care composition" is meant a product, which in the ordinary course of usage, is not intentionally swallowed for purposes of systemic administration of particular therapeutic agents, but is rather retained in the oral cavity for a time sufficient to contact substantially all of the dental surfaces and/or oral tissues for purposes of oral activity. The oral care composition of the present invention is in the form of a mouthrinse,

The term "dispenser", as used herein, means any pump, tube, or container suitable for dispensing oral care compositions.

The term "teeth", as used herein, refers to natural teeth as well as artificial teeth or dental prosthesis.

The term "orally acceptable carrier or excipients" includes materials safe and effective materials and conventional additives used in oral care compositions including but not limited to fluoride ion sources, anti-calculus or anti-tartar agents, buffers, abrasives such as silica, alkali metal bicarbonate salts, thickening materials, humectants, water, surfactants, titanium dioxide, flavor system, sweetening agents, xylitol, coloring agents, and mixtures thereof.

Active and other ingredients useful herein may be categorized or described herein by their cosmetic and/or therapeutic benefit or their postulated mode of action or function. However, it is to be understood that the active and other ingredients useful herein can, in some instances, provide more than one cosmetic and/or therapeutic benefit or function or operate via more than one mode of action. Therefore, classifications herein are made for the sake of convenience and are not intended to limit an ingredient to the particularly stated application or applications listed.

Herein, the terms "tartar" and "calculus" are used interchangeably and refer to mineralized dental plaque biofilms.

The essential and optional components of the present compositions are described in the following paragraphs.

### Tooth Substantive Agent

The present invention includes a tooth substantive agent as an essential ingredient. Suitable tooth substantive agents include polymeric surface active agents (PMSA's). The PMSA's contain anionic groups, i.e., phosphate, and thus, have the capability to interact with cationic or positively charged entities. The "mineral" descriptor is intended to convey that the surface activity or substantivity of the polymer is toward mineral surfaces such as calcium phosphate minerals or teeth.

It is believed the PMSA's provide a stain prevention benefit because of their reactivity or substantivity to mineral surfaces, resulting in desorption of portions of undesirable adsorbed pellicle proteins, in particular those associated with binding color bodies that stain teeth, calculus development and attraction of undesirable microbial species. The retention of these PMSA's on teeth can also prevent stains from accruing due to disruption of binding sites of color bodies on tooth surfaces.

The ability of PMSA's to bind stain promoting ingredients of oral care products such as stannous ions and cationic antimicrobials is also believed to be helpful. The PMSA will also provide tooth surface conditioning effects which produce desirable effects on surface thermodynamic properties and surface film properties, which impart improved clean feel aesthetics both during and most importantly, following rinsing. Many of these polymeric agents are also known or expected to provide tartar control benefits when applied in oral compositions, hence providing improvement in both the appearance of teeth and their tactile impression to consumers.

The desired surface effects include: 1) creating a hydrophilic tooth surface immediately after treatment; and 2) maintaining surface conditioning effects and control of pellicle film for extended periods following product use, including post rinsing and throughout more extended periods. The effect of creating an increased hydrophilic surface can be measured in terms of a relative decrease in water contact angles. The hydrophilic surface, importantly, is maintained on the tooth surface for an extended period after using the product.

The polymeric mineral surface active agents include any agent which will have a strong affinity for the tooth surface, deposit a polymer layer or coating on the tooth surface and produce the desired surface modification effects. The polymers are condensed phosphorylated polymers;

A PMSA according to the invention is a linear polyphosphate. The polyphosphates according to claim 1 are those having from 6 to 21 phosphate groups so that surface adsorption at effective concentrations produces sufficient non-bound phosphate functions, which enhance the anionic surface charge as well as hydrophilic character of the surfaces. Polyphosphates larger than tetrapolyphosphate usually occur as amorphous glassy materials. The polyphosphates in this invention are the linear polyphosphates having the formula:

XO(XPO₃)ₙX

wherein X is sodium, potassium or ammonium and n averages from 6 to 21, such as those commercially known as Sodaphos (n≈6), Hexaphos (n≈13), and Glass H (n≈21) and manufactured by FMC Corporation and Astaris. These polyphosphates may be used alone or in a combination thereof. It is known that polyphosphates are susceptible to hydrolysis in high water formulations at acid pH, particularly below pH 5. Thus it is preferred to use longer-chain polyphosphates, in particular Glass H with an average chain length of 21. It is believed such longer-chain polyphosphates when undergoing hydrolysis produce shorter-chain polyphosphates which are still effective to deposit onto teeth and provide a stain preventive benefit.

Other polyphosphorylated compounds may be used in addition to or instead of the polyphosphate, namely polyphosphorylated inositol compounds such as phytic acid, myo-inositol pentakis(dihydrogen phosphate), myo-inositol tetrakis(dihydrogen phosphate), myo-inositol trikis(dihydrogen phosphate), and an alkali metal, alkaline earth metal or ammonium salt thereof. Preferred herein is phytic acid, also known as myo-inositol 1,2,3,4,5,6-hexakis (dihydrogen phosphate) or inositol hexaphosphoric acid, and its alkali metal, alkaline earth metal or ammonium salts. Herein, the term "phytate" includes phytic acid and its salts as well as the other polyphosphorylated inositol compounds.

The amount of tooth substantive agent required is an effective amount to provide the stain prevention effect. In mouthrinse compositions, the amount of tooth substantive agent is from 0.1% to 5% and more preferably from 0.5% to 3%.

In addition to creating the surface modifying effects, the tooth substantive agent may function to solubilize insoluble salts. For example, Glass H has been found to solubilize insoluble stannous salts. Thus, in compositions containing stannous fluoride for example, Glass H contributes to decreasing the stain promoting effect of stannous.

### Tooth Bleaching Active

The second essential component in the oral composition is a tooth bleaching active. The actives suitable for bleaching are selected from the group consisting of peroxides, perborates, percarbonates, peroxyacids, persulfates, and combinations thereof. Suitable peroxide compounds include hydrogen peroxide, urea peroxide, calcium peroxide, sodium peroxide, zinc peroxide and mixtures thereof. A preferred percarbonate is sodium percarbonate. Preferred persulfates are oxones.

Hydrogen peroxide and urea peroxide are preferred for use in mouthrinse formulations. The following amounts represent the amount of peroxide raw material, although the peroxide source may contain ingredients other than the peroxide raw material. The present composition contains from 0.01%, to 10%, and more preferably from 0.5% to 5% of a peroxide source, by weight of the composition.

In addition to whitening, the peroxide also provides other benefits to the oral cavity. It has long been recognized that hydrogen peroxide and other peroxygen-compounds are effective in curative and/or prophylactic treatments with respect to caries, dental plaque, gingivitis, periodontitis, mouth odor, recurrent aphthous ulcers, denture irritations, orthodontic appliance lesions, postextraction and postperiodontal surgery, traumatic oral lesions and mucosal infections, herpetic stomatitis and the like. Peroxide-containing agents in the oral cavity exert a chemomechanical action generating thousands of tiny oxygen bubbles produced by interaction with tissue and salivary enzymes. The swishing action of a mouthrinse enhances this inherent chemomechanical action. Such action has been recommended for delivery of other agents into infected gingival crevices. Peroxide mouthrinses thus prevent colonization and multiplication of anaerobic bacteria known to be associated with periodontal disease. However, compositions containing hydrogen peroxide or other peroxide releasing compounds generally provide disagreeable taste and mouth sensations, commonly described as stinging, prickling and irritating. In addition peroxides interact with other common excipients therein and tend to be unstable in storage, continuously losing the capacity to release active or nascent oxygen over relatively short periods of time, and tend to diminish or destroy the desired function of such excipients. Among such excipients are flavors, sensory materials and coloring agents added to enhance the acceptability of the oral care product to those in need of an oral peroxidizing or bleaching treatment. The present compositions are surprisingly stable with respect to alteration in flavor profile during storage.

In addition to the components described above, the present compositions may comprise additional optional components collectively referred to as orally acceptable carrier materials, which are described in the following paragraphs.

### Orally Acceptable Carrier Materials

The orally acceptable carrier comprises one or more compatible solid or liquid excipients or diluents which are suitable for topical oral administration. By "compatible," as used herein, is meant that the components of the composition are capable of being commingled without interaction in a manner which would substantially reduce the composition's stability and/or efficacy.

The carriers or excipients of the present invention can include the usual and conventional components of mouthwashes or rinses, as more fully described hereinafter.

Mouthwash or rinse carrier materials typically include water, flavoring and sweetening agents, etc., as disclosed in, e.g., U.S. Pat. No. 3,988,433 to Benedict. preparation of compositions of the present invention are well known in the art. Their selection will depend on secondary considerations like taste, cost, and shelf stability, etc.

The embodiments of the subject invention are mouthwashes or rinses, Components of such mouth rinses typically include one or more of water (from 45% to 95%), ethanol (from 0% to 25%), a humectant (from 0% to 50%), a surfactant (from 0.01 % to 7%), a flavoring agent (from 0.04% to 2%), a sweetening agent (from 0.1% to 3%), and a coloring agent (from 0.001 % to 0.5%). Such mouthrinses may also include one or more of an anticaries agent (from 0.05% to 0.3% as fluoride ion) and an anticalculus agent (from 0.1% to 3%). Components of dental solutions generally include one or more of water (from 90% to 99%), preservative (from 0.01% to 0.5%), thickening agent (from 0% to 5%), flavoring agent (from 0.04% to 2%), sweetening agent (from 0.1% to 3 %), and surfactant ( 0% to 5%).

Types of orally acceptable carriers or excipients which may be included in compositions, along with specific non-limiting examples, are discussed in the following paragraphs.

### Fluoride Source

It is common to have a water-soluble fluoride compound present in oral compositions in an amount sufficient to give a fluoride ion concentration in the composition, and/or when it is used of from 0.0025% to 5.0% by weight, preferably from 0.005% to 2.0% by weight, to provide anticaries effectiveness. A wide variety of fluoride ion-yielding materials can be employed as sources of soluble fluoride in the present compositions. Examples of suitable fluoride ion-yielding materials are found in U.S. Patent No. 3,535,421, October 20, 1970 to Briner et al. and U.S. Patent No. 3,678,154, July 18, 1972 to Widder et al. Representative fluoride ion sources include: stannous fluoride, sodium fluoride, potassium fluoride, sodium monofluorophosphate, indium fluoride and many others. Stannous fluoride and sodium fluoride are preferred, as well as mixtures thereof.

### Anticalculus Agent

The present compositions may optionally include an additional anticalculus agent, such as a pyrophosphate salt as a source of pyrophosphate ion. The pyrophosphate salts useful in the present compositions include the dialkali metal pyrophosphate salts, tetraalkali metal pyrophosphate salts, and mixtures thereof. Disodium dihydrogen pyrophosphate (Na₂H₂P₂O₇), tetrasodium pyrophosphate (Na₄P₂O₇), and tetrapotassium pyrophosphate (K₄P₂O₇) in their unhydrated as well as hydrated forms are the preferred species. In compositions, the pyrophosphate salt may be present in one of three ways: predominately dissolved, predominately undissolved, or a mixture of dissolved and undissolved pyrophosphate.

Compositions comprising predominately dissolved pyrophosphate refer to compositions where at least one pyrophosphate ion source is in an amount sufficient to provide at least about 1.0% free pyrophosphate ions. The amount of free pyrophosphate ions may be from about 1% to about 15%, from about 1.5% to about 10% in one embodiment, and from about 2% to about 6% in another embodiment. Free pyrophosphate ions may be present in a variety of protonated states depending on the pH of the composition.

Compositions comprising predominately undissolved pyrophosphate refer to compositions containing no more than about 20% of the total pyrophosphate salt dissolved in the composition, preferably less than about 10% of the total pyrophosphate dissolved in the composition. Tetrasodium pyrophosphate salt is a preferred pyrophosphate salt in these compositions. Tetrasodium pyrophosphate may be the anhydrous salt form or the decahydrate form, or any other species stable in solid form in the compositions. The salt is in its solid particle form, which may be its crystalline and/or amorphous state, with the particle size of the salt preferably being small enough to be aesthetically acceptable and readily soluble during use. The amount of pyrophosphate salt useful in making these compositions is any tartar control effective amount, generally from about 1.5% to about 15%, preferably from about 2% to about 10%, and most preferably from about 3% to about 8%, by weight of the composition.

Compositions may also comprise a mixture of dissolved and undissolved pyrophosphate salts. Any of the above mentioned pyrophosphate salts may be used.

The pyrophosphate salts are described in more detail in Kirk-Othmer Encyclopedian, of Chemical Technology, Third Edition, Volume 17, Wiley-Interscience Publishers (1982).

Optional agents to be used in place of or in combination with the pyrophosphate salt include such known materials as synthetic anionic polymers, including polyacrylates and copolymers of maleic anhydride or acid and methyl vinyl ether (e.g., Gantrez), as described, for example, in U.S. Patent 4,627,977, to Gaffar et al., as well as, e.g., polyamino propane sulfonic acid (AMPS), diphosphonates (e.g., EHDP; AHP), polypeptides (such as polyaspartic and polyglutamic acids), and mixtures thereof.

### Chelating agents

Another optional agent is a chelating agent, also called sequestrants, such as gluconic acid, tartaric acid, citric acid and pharmaceutically-acceptable salts thereof. Chelating agents are able to complex calcium found in the cell walls of the bacteria. Chelating agents can also disrupt plaque by removing calcium from the calcium bridges which help hold this biomass intact. However, it is not desired to use a chelating agent which has an affinity for calcium that is too high, as this may result in tooth demineralization, which is contrary to the objects and intentions of the present invention. Suitable chelating agents will generally have a calcium binding constant of about 10¹ to 10⁵ to provide improved cleaning with reduced plaque and calculus formation. Chelating agents also have the ability to complex with metallic ions and thus aid in preventing their adverse effects on the stability or appearance of products. Chelation of ions, such as iron or copper, helps retard oxidative deterioration of finished products.

Examples of suitable chelating agents are sodium or potassium gluconate and citrate; citric acid/alkali metal citrate combination; disodium tartrate; dipotassium tartrate; sodium potassium tartrate; sodium hydrogen tartrate; potassium hydrogen tartrate and mixtures thereof. The amounts of chelating agent suitable are 0.1% to 2.5%, preferably from 0.5% to 2.5% and more preferably from 1.0% to 2.5%.

Still other chelating agents suitable are the anionic polymeric polycarboxylates. Such materials are well known in the art, being employed in the form of their free acids or partially or preferably fully neutralized water soluble alkali metal (e.g. potassium and preferably sodium) or ammonium salts. Examples are 1:4 to 4:1 copolymers of maleic anhydride or acid with another polymerizable ethylenically unsaturated monomer, preferably methyl vinyl ether (methoxyethylene) having a molecular weight (M.W.) of 30,000 to 1,000,000. These copolymers are available for example as Gantrez AN 139 (M.W. 500,000), AN 119 (M.W. 250,000) and S-97 Pharmaceutical Grade (M.W. 70,000), of GAF Chemicals Corporation.

Other operative polymeric polycarboxylates include the 1:1 copolymers of maleic anhydride with ethyl acrylate, hydroxyethyl methacrylate, N-vinyl-2-pyrrolidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone.

Additional operative polymeric polycarboxylates are disclosed in U.S. Patent 4,138,477, February 6, 1979 to Gaffar and U.S. Patent 4,183,914, January 15, 1980 to Gaffar et al. and include copolymers of maleic anhydride with styrene, isobutylene or ethyl vinyl ether; polyacrylic, polyitaconic and polymaleic acids; and sulfoacrylic oligomers of M.W. as low as 1,000 available as Uniroyal ND-2.

### Other Active Agents

The composition may optionally include other agents, such as antimicrobial agents. Included among such agents are water insoluble non-cationic antimicrobial agents such as halogenated diphenyl ethers, phenolic compounds including phenol and its homologs, mono and poly-alkyl and aromatic halophenols, resorcinol and its derivatives, bisphenolic compounds and halogenated salicylanilides, benzoic esters, and halogenated carbanilides. The water soluble antimicrobials include quaternary ammonium salts and bis-biquanide salts, and triclosan monophosphate. The quaternary ammonium agents include those in which one or two of the substitutes on the quaternary nitrogen has a carbon chain length (typically alkyl group) from 8 to 20, typically from 10 to 18 carbon atoms while the remaining substitutes (typically alkyl or benzyl group) have a lower number of carbon atoms, such as from 1 to 7 carbon atoms, typically methyl or ethyl groups. Dodecyl trimethyl ammonium bromide, tetradecylpyridinium chloride, domiphen bromide, N-tetradecyl-4-ethyl pyridinium chloride, dodecyl dimethyl (2-phenoxyethyl) ammonium bromide, benzyl dimethylstearyl ammonium chloride, cetyl pyridinium chloride, quaternized 5-amino-1,3-bis(2-ethylhexyl)-5-methyl hexa hydropyrimidine, benzalkonium chloride, benzethonium chloride and methyl benzethonium chloride are exemplary of typical quaternary ammonium antibacterial agents. Other compounds are bis[4-(R-amino)-1-pyridinium] alkanes as disclosed in U.S. Patent 4,206,215, issued June 3, 1980, to Bailey. Other antimicrobials such as copper salts, zinc salts and stannous salts may also be included. Also useful are enzymes, including endoglycosidase, papain, dextranase, mutanase, and mixtures thereof. Such agents are disclosed in U.S. Patent 2,946,725, Jul. 26, 1960, to Norris et al. and in U.S. Patent 4,051,234, September. 27, 1977 to Gieske et al. Preferred antimicrobial agents include zinc salts, stannous salts, cetyl pyridinium chloride, chlorhexidine, triclosan, triclosan monophosphate, and flavor oils such as thymol. Triclosan and other agents of this type are disclosed in Parran, Jr. et al., U.S. Patent 5,015,466, issued May 14, 1991, and U.S. Patent 4,894,220, Jan. 16, 1990 to Nabi et al. These agents provide anti-plaque benefits and are typically present at levels of from about 0.01% to about 5.0%, by weight of the composition.

Another optional active agent that may be added to the present compositions is a dentinal desensitizing agent to control hypersensitivity, such as salts of potassium, calcium, strontium and tin including nitrate, chloride, fluoride, phosphates, pyrophosphate, polyphosphate, citrate, oxalate and sulfate.

### Surfactants

The present compositions may also comprise surfactants, also commonly referred to as sudsing agents. Suitable surfactants are those which are reasonably stable and foam throughout a wide pH range. The surfactant may be anionic, nonionic, amphoteric, zwitterionic, cationic, or mixtures thereof.

Anionic surfactants useful herein include the water-soluble salts of alkyl sulfates having from 8 to 20 carbon atoms in the alkyl radical (e.g., sodium alkyl sulfate) and the water-soluble salts of sulfonated monoglycerides of fatty acids having from 8 to 20 carbon atoms. Sodium lauryl sulfate (SLS) and sodium coconut monoglyceride sulfonates are examples of anionic surfactants of this type. Other suitable anionic surfactants are sarcosinates, such as sodium lauroyl sarcosinate, taurates, sodium lauryl sulfoacetate, sodium lauroyl isethionate, sodium laureth carboxylate, and sodium dodecyl benzenesulfonate. Mixtures of anionic surfactants can also be employed. Many suitable anionic surfactants are disclosed by Agricola et al., U.S. Patent 3,959,458, issued May 25, 1976. The composition typically comprises an anionic surfactant at a level of from 0.025% to 9%, from 0.05% to 5% in some embodiments, and from 0.1% to 1% in other embodiments.

Another suitable surfactant is one selected from the group consisting of sarcosinate surfactants, isethionate surfactants and taurate surfactants. Preferred for use herein are alkali metal or ammonium salts of these surfactants, such as the sodium and potassium salts of the following: lauroyl sarcosinate, myristoyl sarcosinate, palmitoyl sarcosinate, stearoyl sarcosinate and oleoyl sarcosinate. The sarcosinate surfactant may be present in the compositions from 0.1% to 2.5%, preferably from 0.5% to 2.0% by weight of the total composition.

Useful cationic surfactants include derivatives of aliphatic quaternary ammonium compounds having one long alkyl chain containing from about 8 to 18 carbon atoms such as lauryl trimethylammonium chloride; cetyl pyridinium chloride; cetyl trimethylammonium bromide; di-isobutylphenoxyethyl-dimethylbenzylammonium chloride; coconut alkyltrimethylammonium nitrite; cetyl pyridinium fluoride; etc. Preferred compounds are the quaternary ammonium fluorides described in U.S. Patent 3,535,421, October 20, 1970, to Briner et al., where said quaternary ammonium fluorides have detergent properties. Certain cationic surfactants can also act as germicides in the compositions disclosed herein. Cationic surfactants such as chlorhexidine, although suitable for use in the current composition, are not preferred due to their capacity to stain the oral cavity's hard tissues. Persons skilled in the art are aware of this possibility and should incorporate cationic surfactants only with this limitation in mind.

Nonionic surfactants that can be used in the compositions include compounds produced by the condensation of alkylene oxide groups (hydrophilic in nature) with an organic hydrophobic compound which may be aliphatic or alkylaromatic in nature. Examples of suitable nonionic surfactants include the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

Useful zwitterionic synthetic surfactants include derivatives of aliphatic quaternary ammonium, phosphonium, and sulfonium compounds, in which the aliphatic radicals can be straight chain or branched, and wherein one of the aliphatic substituents contains from 8 to 18 carbon atoms and one contains an anionic water-solubilizing group, e.g., carboxy, sulfonate, sulfate, phosphate or phosphonate.

Suitable betaine surfactants are disclosed in U.S. Patent 5,180,577 to Polefka et al., issued January 19, 1993. Typical alkyl dimethyl betaines include decyl betaine or 2-(N-decyl-N,N-dimethylammonio) acetate, coco betaine or 2-(N-coc-N, N-dimethyl ammonio) acetate, myristyl betaine, palmityl betaine, lauryl betaine, cetyl betaine, cetyl betaine, stearyl betaine, etc. The amidobetaines are exemplified by cocoamidoethyl betaine, cocoamidopropyl betaine, lauramidopropyl betaine and the like. The betaines of choice are preferably the cocoamidopropyl betaine and, more preferably, the lauramidopropyl betaine.

### Humectants

Another optional carrier material of the present compositions is a humectant. The humectant serves to give compositions a moist feel to the mouth, and, for particular humectants, to impart desirable sweetness of flavor to compositions. The humectant, on a pure humectant basis, generally comprises from 0% to 70%, preferably from about 5% to about 25%, by weight of the compositions herein. Suitable humectants for use in compositions of the subject invention include edible polyhydric alcohols such as glycerin, sorbitol, xylitol, butylene glycol, polyethylene glycol, propylene glycol and trimethyl glycine.

### Flavoring and Sweetening Agents

Flavoring agents may also be added to the compositions. However, many of the typical flavoring agents are chemically incompatible with bleaching agents, in particular peroxide. Examples of suitable flavoring agents as disclosed in U.S. Patent No. 4,684,517 to Clipper et al. include menthol, methyl salicylate, cinnamic aldehyde and clove oil. Flavoring agents are generally used in the compositions at levels of from 0. 00 1 % to 5%, by weight of the composition.

Sweetening agents which can be used include sucrose, glucose, saccharin, sucralose, dextrose, levulose, lactose, mannitol, sorbitol, fructose, maltose, xylitol, saccharin salts, thaumatin, aspartame, D-tryptophan, dihydrochalcones, acesulfame and cyclamate salts, especially sodium cyclamate, sucralose and sodium saccharin, and mixtures thereof. A composition preferably contains from 0.1 % to 10% of these agents, preferably from 0.1% to 1%, by weight of the composition.

In addition to flavoring and sweetening agents, coolants, salivating agents, warming agents, and numbing agents can be used as optional ingredients in the compositions . These agents are present in the compositions at a level of from 0.001% to 10%, preferably from 0.1% to 1%, by weight of the composition.

The coolant can be any of a wide variety of materials. Included among such materials are carboxamides, menthol, ketals, diols, and mixtures thereof. Preferred coolants in the present compositions are the paramenthan carboxyamide agents such as N-ethyl-p-menthan-3-carboxamide, known commercially as "WS-3", N,2,3-trimethyl-2-isopropylbutanamide, known as "WS-23," and mixtures thereof. Additional preferred coolants are selected from the group consisting of menthol, 3-1-menthoxypropane-1,2-diol known as TK-10 manufactured by Takasago, menthone glycerol acetal known as MGA manufactured by Haarmann and Reimer, and menthyl lactate known as Frescolat® manufactured by Haarmann and Reimer. The terms menthol and menthyl as used herein include dextro- and levorotatory isomers of these compounds and racemic mixtures thereof. TK-10 is described in U.S. Pat. No. 4,459,425, Amano et al., issued 7/10/84. WS-3 and other agents are described in U.S. Pat. No. 4,136,163, Watson, et al., issued Jan. 23, 1979.

Suitable salivating agents include Jambu® manufactured by Takasago. Examples of warming agents are capsicum and nicotinate esters, such as benzyl nicotinate. Suitable numbing agents include benzocaine, lidocaine, clove bud oil, and ethanol.

### Miscellaneous Carrier Materials

Water employed in the preparation of commercially suitable oral compositions should preferably be of low ion content and free of organic impurities. Water generally comprises up to 99% by weight of the aqueous compositions herein. These amounts of water include the free water which is added plus that which is introduced with other materials, such as with sorbitol.

The composition may also include an alkali metal bicarbonate salt, which may serve a number of functions including abrasive, deodorant, buffering and adjusting pH. Alkali metal bicarbonate salts are soluble in water and unless stabilized, tend to release carbon dioxide in an aqueous system. Sodium bicarbonate, also known as baking soda, is a commonly used alkali metal bicarbonate salt. The composition may contain from 0.5% to 30%, preferably from 0.5% to 15%, and most preferably from 0.5% to 5% of an alkali metal bicarbonate salt.

The pH of the present compositions may be adjusted through the use of buffering agents. Buffering agents, as used herein, refer to agents that can be used to adjust the pH of aqueous mouthrinses to a range of pH 4.0 to pH 6.0 for peroxide stability. Buffering agents include sodium bicarbonate, monosodium phosphate, trisodium phosphate, sodium hydroxide, sodium carbonate, sodium acid pyrophosphate, citric acid, and sodium citrate. Buffering agents are typically included at a level of from 0.5% to 10%, by weight of the present compositions.

Poloxamers may be employed in the present compositions. A poloxamer is classified as a nonionic surfactant and may also function as an emulsifying agent, binder, stabilizer, and other related functions. Poloxamers are difunctional block-polymers terminating in primary hydroxyl groups with molecular weights ranging from 1,000 to above 15,000. Poloxamers are sold under the tradename of Pluronics and Pluraflo by BASF. Suitable poloxamers for this invention are Poloxamer 407 and Pluraflo L4370.

Other emulsifying agents that may be used in the present compositions include polymeric emulsifiers such as the Pemulen® series available from B.F. Goodrich, and which are predominantly high molecular weight polyacrylic acid polymers useful as emulsifiers for hydrophobic substances.

Titanium dioxide may also be added to the present composition. Titanium dioxide is a white powder which adds opacity to the compositions. Titanium dioxide generally comprises from about 0.25% to about 5% by weight of dentifrice compositions.

Other optional agents that may be used in the present compositions include dimethicone copolyols selected from alkyl- and alkoxy-dimethicone copolyols, such as C 12 to C20 alkyl dimethicone copolyols and mixtures thereof. Highly preferred is cetyl dimethicone copolyol marketed under the trade name Abil EM90. The dimethicone copolyol is generally present in a level of from about 0.01% to about 25%, preferably froom about 0.1% to about 5%, more preferably from about 0.5% to about 1.5% by weight. The dimethicone copolyols aid in providing positive tooth feel benefits.

### Method of Use

For whitening teeth and preventing staining. a subject's dental enamel surfaces and mucosa in the mouth should be contacted with the oral compositions according to the present invention. contacting may be rinsing with mouthrinse. The subject may be any person or animal whose tooth surface contact the oral composition. By animal is meant to include household pets or other domestic animals, or animals kept in captivity.

Example would include the rinsing of a cat's mouth with an oral composition for a sufficient amount of time to see a benefit.

### EXAMPLES -

The following examples further describe and demonstrate embodiments within the scope of the present invention. These examples are given solely for the purpose of illustration and are not to be construed as limitations of the present invention as many variations thereof are possible.

### Example I Mouthrinse Compositions

Mouthrinse compositions according to the present invention (IA - IE) are shown below with amounts of components in weight %. These compositions are made using conventional methods.

| Ingredient | Ex. IA | Ex. IB | Ex. IC | Ex. ID | Ex. IE |
|---|---|---|---|---|---|
| | % W/W | % W/W | % W/W | % W/W | % W/W |
| Hydrogen Peroxide¹ | 0.5 | 0.5 | 1.5 | 3.0 | 3.0 |
| Polyphosphate² | 0.2 | 2.0 | 1.0 | 0.5 | 2.0 |
| Flavor | 0.2 | 0.2 | 0.2 | 0.2 | 0.2 |
| Poloxamer 407 | 0.75 | 0.75 | 0.75 | 1.0 | 1.0 |
| Glycerin | 15.0 | 10.0 | 11.0 | 5.0 | 5.0 |
| Propylene Glycol | 3.0 | 3.0 | 3.0 | 3.0 | 3.0 |
| Sucralose | 0.05 | 0.05 | --- | --- | |
| Sodium Saccharin | --- | --- | 0.06 | 0.06 | 0.06 |
| Water | QS | QS | QS | QS | QS |

Hydrogen Peroxide added as 35% solution (Amount shown above is actual peroxide level in composition.)

Polyphosphate is Glass H supplied by Astaris.

### Example II Stain Prevention Effects

The stain prevention effects of the present compositions are measured using an *in* vitro method. Hydroxyapatite (HAP) powder was weighed (200 ± 10 mg) and exposed to treatment solutions (neat oral rinses) for 30 seconds. HAP was then removed by centrifugation (15,000 rpm for 15 minutes), washed by re-suspension in deionized water, and removed by centrifugation. The centrifugation/re-suspension step was repeated. Staining of the HAP powder was carried out by adding the treated and washed HAP powder to freshly brewed tea (1 bag/100ml, 50°C) for 30 seconds followed by washing twice. Washed HAP was re-suspended in 10 ml water and passed through vacuum filtration using a 0.45 µm pore filter. This was dried and laminated. Color readings of the HAP were taken using digital photography using the white light imaging system (Fuji 2000 Camera). Color values (L*a*b*) were determined using Giant 2.0.1 software package. L* represents lightness on the y axis, a* represents chroma (red-green) on the x axis, and b* represents chroma (yellow-blue) on the z axis. Changes in the individual L*, a*, and b* components (Δ values) were calculated by subtracting the L*a*b* measurements of treated HAP powder from the L*a*b* measurements of untreated and unstained HAP powder. The total color change (ΔE) was calculated as the square root of the sum of the square of the Δ values. Results are summarized in the Table 1 below showing the total change in Lightness ΔL*, change in yellowness Δb* and Total Color ΔE. The present composition prevented staining significantly better than negative controls (water and Crest Cavity Protection) and about the same degree as Crest Dual Action Whitening, which contains polyphosphate. There was no difference in performance between product as made compared to product after aging at 40 °C, demonstrating that the efficacy of the product is maintained during storage at accelerated conditions.

**Table 1. Results of Stain Prevention Testing**

| Treatment Product | ΔL* | Δb* | ΔE |
|---|---|---|---|
| Water | 33.68 | 18.32 | 39.93 |
| Example IC | 0.29 | 0.012 | 0.29 |
| Example 1C aged @ 40 °C | 0.29 | 0.014 | 0.29 |
| Crest Cavity Protection | 22.60 | 17.04 | 29.27 |
| Crest Dual Action Whitening | 0.22 | 0.084 | 0.24. |

### Example III Stability of Compositions

The stability of the present compositions was assessed by measuring peroxide level, pH and hydrolysis of the polyphosphate under storage conditions at 25 °C and 40 °C.

Hydrogen peroxide was measured using an aqueous compatible PeroXOquant™ quantitative peroxide assay which detects peroxide based on the oxidation of ferrous to ferric ion in the presence of xylenol orange. Peroxide first reacts with sorbitol, converting it to a peroxyl radical, which in turn initiates the Fe²⁺ oxidation to Fe³⁺. The Fe³⁺ complexes with the xylenol orange dye to produce a purple product. This complex is measured using a microplate spectrophotometer at a wavelength of 595 nm to determine the hydrogen peroxide in the sample. The method has a margin of error up to about 10%.

The pH of Example IC mouthrinse product was measured using a potentiometric instrument (Orion Model 720A) and an Orion PerpHect® combination pH electrode at room temperature.

Hydrolysis of the polyphosphate was measured by the level of orthophosphate (n=1) and trimetaphosphate (n=3 cyclic), that are split off from linear polyphosphate chains, leaving the chain shortened by n=1 or n=3, respectively. Increasing levels of orthophosphate and trimetaphosphate indicate the degree of hydrolysis of the polyphosphate.

Results of stability testing are summarized in Table 2 below indicating that the present composition is stable against loss of peroxide during storage at 25 °C and 40 °C. The pH tended to decrease during storage and polyphosphate hydrolysis increased with the drop in pH. However as demonstrated in Example II, no change in stain prevention efficacy is observed with aged product.

**Table 2. Results of Stability Testing**

| Storage Temperature | | 25°C | | 40 °C | |
|---|---|---|---|---|---|
| Storage Time, Months | 0 | 1 | 3 | 1 | 3 |
| H₂O₂, wt. % | 1.37% | 1.39% | 1.47% | 1.38% | 1.42% |
| pH | 5.44 | 5.36 | 5.25 | 5.08 | 4.72 |
| Orthophosphate, wt. % | 1.15% | 2.30% | 3.00% | 8.80% | 17.9% |
| Trimetaphosphate, wt.% | 4.63% | 8.03% | 9.97% | 23.1% | 33.4% |

### Reference example IV Dual-Phase Dentifrice Compositions

Dual phase dentifrice compositions according to the present invention are comprised of a first dentifrice composition (IVA - IVC) and a second dentifrice composition (IVD -IVE), preferably at a 50:50 ratio. These compositions are made using conventional methods.

| Ingredient | First Dentifrice | | | Second Dentifrice | | |
|---|---|---|---|---|---|---|
| | IVA | IVB | IVC | IVD | IVE | IVF |
| Glass H Polyphosphate | 7.00 | 7.00 | | | | |
| Sodaphos Polyphosphate | | | 7.00 | | | |
| Calcium Peroxide | 1.00 | 0.40 | 5.00 | | | |
| Sodium Fluoride | | | | 0.486 | 0.486 | |
| Stannous Fluoride | | | | | 0.908 | 0.908 |
| Stannous Chloride | | | | | | 3.000 |
| Sodium Gluconate | | | | | | 4.160 |
| Carboxymethycellulose | 0.60 | 0.60 | 0.60 | | | |
| Carbomer | | | | | 0.20 | |
| Water | 7.00 | 7.00 | 5.00 | 33.00 | 25.10 | 21.840 |
| Flavor | 1.00 | 1.00 | 1.00 | 0.40 | 0.90 | 1.00 |
| Color | | | | 0.30 | 0.40 | 0.30 |
| Glycerin | 43.20 | 26.80 | 24.20 | 44.514 | 9.00 | 28.992 |
| Sorbitol | | | | | 29.594 | |
| Poloxamer 407 | 5.00 | 5.00 | 5.00 | 21.00 | | 15.500 |
| Polyethylene Glycol | 3.00 | 3.00 | 3.00 | | 3.00 | |
| Propylene Glycol | 5.00 | 5.00 | 5.00 | | | |
| Sodium Alkyl Sulfate^{(a)} | 4.00 | 4.00 | 4.00 | | 4.00 | |
| Silica | 20.00 | 22.00 | 22.00 | | 22.50 | 23.000 |
| Sodium Hydroxide ^{(b)} | | | | | | 1.000 |
| Sodium Bicarbonate | | 15.00 | 15.00 | | | |
| Sodium Carbonate | 2.00 | 2.00 | 2.00 | | | |
| Sodium Saccharin | 0.50 | 0.50 | 0.50 | 0.30 | 0.50 | 0.30 |
| Titanium Dioxide | 0.50 | 0.50 | 0.50 | | | |
| Xanthan Gum | 0.20 | 0.20 | 0.20 | | 0.60 | |
| Sodium Acid Pyrophosphate | | | | | 0.50 | |
| Tetrasodium Pyrophosphate | | | | | 3.22 | |

| | | | | | | |
|---|---|---|---|---|---|---|
| ^{(a)} 27.9% solution ^{(b)} 50% solution | | | | | | |

## Claims

1. An oral care composition in the form of a mouthrinse providing enhanced tooth whitening and stain prevention comprising
(a) from 0.01% to 10%, by weight of the composition, of a peroxide tooth bleaching active;
(b) from 0.1% to 5% by weight of the composition of a polyphosphorylated compound selected from linear polyphosphates having an average chain length of 6 to 21, phytic acid, myo-inositol pentakis(dihydrogen phosphate), myo-inositol tetrakis(dihydrogen phosphate), myo-inositol trikis(dihydrogen phosphate), alkali metal, alkaline earth metal or ammonium salts thereof and mixtures thereof,
(c) from 45% - 95% by weight of the composition water and
(d) an orally-acceptable aqueous carrier,
wherein the composition has a pH ranging from 4.0 to 6.0 and wherein the composition is stable against loss of peroxide for at least three months under storage conditions at 40 °C.

2. An aqueous composition according to Claim 1 comprising hydrogen peroxide or urea peroxide as bleaching agent and a linear polyphosphate having an average chain length of from 6 to 21.

3. An oral care composition according to Claim 1 comprising a flavor component comprising one or a mixture of cooling agents selected from menthol, carboxamides, ketals, diols, and mixtures thereof.

## Patentansprüche

1. Mundpflegezusammensetzung in der Form einer Mundspülung, die verbesserte Zahnaufhellung und Fleckenvorbeugung bereitstellt, umfassend
(a) zu 0,01 Gew.-% bis 10 Gew.-% der Zusammensetzung einen Peroxid-Zahnbleichungswirkstoff,
(b) zu 0,1 Gew.-% bis 5 Gew.-% der Zusammensetzung eine polyphosphorylierte Verbindung, ausgewählt aus linearen Polyphosphaten mit einer durchschnittlichen Kettenlänge von 6 bis 21, Phytinsäure, Myoinositpentakis(dihydrogenphosphat), Myoinosittetrakis(dihydrogenphosphat), Myoinosittrikis(dihydrogenphosphat), Alkalimetall-, Erdalkalimetall- oder Ammoniumsalze davon und Mischungen davon,
(c) zu 45 Gew.-% bis 95 Gew.-% der Zusammensetzung Wasser und
(d) einen oral unbedenklichen wässrigen Träger,
wobei die Zusammensetzung einen pH im Bereich von 4,0 bis 6,0 aufweist und wobei die Zusammensetzung unter Lagerbedingungen bei 40 °C für mindestens drei Monate gegen Verlust von Peroxid stabil ist.

2. Wässrige Zusammensetzung nach Anspruch 1, umfassend Wasserstoffperoxid oder Harnstoffperoxid als Bleichmittel und ein lineares Polyphosphat mit einer durchschnittlichen Kettenlänge von 6 bis 21.

3. Mundpflegezusammensetzung nach Anspruch 1, umfassend einen Geschmackstoffbestandteil, umfassend eines oder eine Mischung von Kühlmitteln, ausgewählt aus Menthol, Carboxamiden, Ketalen, Diolen und Mischungen davon.

## Revendications

1. Composition de soins bucco-dentaires sous la forme d'un bain de bouche assurant un blanchiment des dents et une prévention des taches améliorés comprenant
(a) de 0,01 % à 10 %, en poids de la composition, d'un actif de blanchiment des dents au peroxyde ;
(b) de 0,1 % à 5 % en poids de la composition d'un composé polyphosphorylé choisi parmi les polyphosphates linéaires ayant une longueur de chaîne moyenne comprise entre 6 et 21, l'acide phytique, le pentakis de myo-inositol (dihydrogénophosphate), le tétrakis de myo-inositol (dihydrogénophosphate), le trikis de myo-inositol (dihydrogénophosphate), un métal alcalin, un métal alcalino-terreux ou leurs sels d'ammonium et leurs mélanges,
(c) de 45 % à 95 % en poids de la composition d'eau, et
(d) un support aqueux acceptable par voie orale,
dans laquelle la composition a un pH allant de 4,0 à 6,0, et dans laquelle la composition est stable par rapport à la perte de peroxyde pendant au moins trois mois dans des conditions de stockage à 40 °C.

2. Composition aqueuse selon la revendication 1, comprenant du peroxyde d'hydrogène ou du peroxyde d'urée comme agent de blanchiment et un polyphosphate linéaire ayant une longueur de chaîne moyenne comprise entre 6 et 21.

3. Composition de soins bucco-dentaires selon la revendication 1, comprenant un composant aromatique comprenant un agent ou un mélange d'agents de rafraîchissement choisis parmi le menthol, les carboxamides, les cétals, les diols et leurs mélanges.
